# EUROPEAN PATENT APPLICATION

(11) **EP 4 465 301 A1**
(43) Date of publication of application: **20.11.2024**
(21) Application number: 23176743.5
(22) Date of filing: 01.06.2023
(51) Int. Cl.: G16H 15/00, G16H 30/40, G16H 50/20

(54) **INTERACTIVE AND EXPLAINABLE CO-DECISION-MAKING REPORTING SYSTEM**

(30) Priority: 17.05.2023 WO PCT/CN2023/202309
(71) Applicant: Koninklijke Philips N.V., 5656 AG Eindhoven (NL)
(72) Inventor: JIN, Peijie, Eindhoven (NL); YU, Wenjin, Eindhoven (NL); JIN, Hua, 5656AG Eindhoven (NL); DENG, Junping, Eindhoven (NL); SU, Bin, Eindhoven (NL); DESHPANDE, Hrishikesh Narayanrao, Eindhoven (NL); BUELOW, Thomas, Eindhoven (NL)
(74) Representative: Philips Intellectual Property & Standards

(57) **Abstract**

A method for clinical decision support is described. The method comprises the steps of obtaining radiology examination data, obtaining clinical information data, determining, by a diagnosis module, at least one object of interest in the clinical information data, and determining, by an interpretability module, at least one region of interest in the radiology examination data. Furthermore, the method maps the clinical information data to the radiology examination data, transmits the result to at least one node in a healthcare network. Clinical feedback from the at least one node in the healthcare network can be obtained, and the clinical report comprising the clinical feedback transmitted and displayed.

## Description

### BACKGROUND OF THE INVENTION

Clinical decision-making has always been a challenge considering the number of different specialists involved, as well as the complexities of the procedures, the clinical workflow and many other associated aspects. This is especially the case if the patient needs to be involved in the clinical decision-making, or if feedback needs to be received from different clinical sites. Furthermore, due to the unequal medical knowledge, various clinical roles, and different cognitions of doctors and patients, there are often differences in medical decision-making, and medical disputes may occur. There exists an information gap among radiologists, clinicians, and patients. For example, with the limited input from clinicians and patients, radiologists only focus on the global examination imaging rather than the specific regions of interest. Furthermore, with the limited support of radiology reports, clinicians sometimes would rather read the imaging study again to make the decision than rely on the reports, which adds additional bottlenecks in the clinical practice.

On the other hand, for the majority of clinical doctors (including general practitioners, young doctors, specialists when dealing with non-specialty diseases, etc.), the time is a very scarce resource. Clinicians cannot do their job properly enough if they don't have access to critical, easy to comprehend and complete information. In many cases this information comes from specialists of different specialities, which in some cases sit in different sites, states or even countries. This is especially critical in cases, where medical imaging devices, such as Computed Tomography (CT) or Magnetic Resonance Imaging (MRI), are involved due to specific data standards that are attributable to these devices. Yet, having timely access to this data is of paramount importance for the correct diagnosis of the patients as clinicians must pay attention to imaging examination, understand the important findings of imaging, even communicate with radiologists on some uncertain cases.

Hence, the communication and feedback workflow in clinical practice needs to be improved.

### SUMMARY OF THE INVENTION

The current invention serves to introduce a method, system and associated computer program/computer-readable medium that improves clinical decision support by sharing of information and facilitating acquiring feedback from different sites of a clinical institution.

The invention is defined by the independent claims. Dependent claims represent beneficial embodiments of the invention.

In one aspect of the invention, a method for clinical decision support is described. The method comprises the steps of: obtaining radiology examination data from at least one of the following modalities: imaging device, Digital Imaging and Communications in Medicine (DICOM) system, Picture Archiving and Communications (PACS) system; obtaining clinical information data from at least one of the following modalities: electronic health record (EHR), electronic medical record (EMR), hospital information system (HIS), radiology information system (RIS), Systematized Nomenclature of Medicine-Clinical Terms (SNOMED-CT); determining, by a diagnosis module, at least one object of interest in the clinical information data, and determining, by an interpretability module, at least one region of interest in the radiology examination data; mapping the clinical information data and/or the at least one object of interest to the radiology examination data and/or at least one region of interest thereby obtaining a data mapping; transmitting the clinical information data, the radiology examination data and/or the data mapping to at least one node in a healthcare network; obtaining a clinical feedback from the at least one node in the healthcare network; providing a clinical report comprising highlighting at least one of: the regions of interest, the objects of interest, the clinical feedback from the at least one node in the healthcare network.

It is to be understood that the radiology examination data could comprise any data obtained from medical imaging devices, which is not necessarily limited to radiology devices. This might include: X-ray, Computed Tomography (CT), Magnetic Resonance Imaging (MRI), Positron Emission Tomography (PET), Mammography, Cone Beam Computed Tomography (CBCT), endoscopy, Ultrasound (US), Fluoroscopy, SPECT, elastography, pathology and other devices producing a medical image. The radiology examination data can be obtained directly from the imaging devices, or from one of the archiving systems, such as PACS, DICOM system, and others. It is to be further understood that clinical information data comprises data that comprises patient information data. This could include structured or unstructured data, and could be obtained from EHR, EMR, HIS, RIS, SNOMED-CT or any other modality that has patient information data that could be useful for clinical diagnosis. It is to be understood that the clinical information data not necessarily resides in the clinical institution, and could include data coming from e.g., an insurance database.

Upon receiving the clinical information data and radiology examination data, the clinical decision support system determines regions or objects of interest. It is to be understood that regions of interest pertain to an image (radiology examination data) from the medical imaging device, whereas objects of interest pertain to structured or unstructured data in the clinical information data. Regions of Interest (ROI) and Objects of Interest (OOI) are identified by the diagnosis and interpretability module. ROI and OOI in some embodiments relates to clinically significant objects of interest based on e.g., clinical guidelines, atlas images, machine learning approaches, prior patients, etc. For example, tumor lesions can be detected based on a machine learning algorithm that analyzes previous images, and determines if a tumor is present by comparison. It is to be understood that this is a non-limiting example, and other examples are possible within the confines of the present invention. When the identification of ROI's and OOI's is done, the system maps the information from any one of: ROI's and radiology examination data to OOI's and clinical information data. To give a non-limiting example, the system detects that a lesion is present, the system also detects the objects of interest in the clinical report (e.g., Increased CTC number), and maps the e.g., increased CTC number to the lesion. This allows the physician to quickly see a link between the clinical information data and the radiology examination data. As a result of the mapping, the mapping data is obtained, which comprises one or more mappings of one or more patients. When the mapping data is obtained, it is transmitted to at least one node of a healthcare network. The nodes of the healthcare network are a collective term, and could include nodes within the same healthcare institution (e.g., the nodes within the radiology department), or nodes outside of the healthcare institution (e.g., remote teleradiology nodes from different countries). The clinical feedback from the nodes is obtained as the next step. In some embodiments this could be an optional step, e.g., if there is no substantial feedback needed, and the person on the receiving side of the nodes merely approves the clinical finding and plan. Hence, the feedback from the nodes could be of confirmatory nature (e.g., the findings are correct, and the clinical specialist approves the findings), or it could be of participatory nature (e.g., the clinical specialist adds additional details to the mapping, such as comments, reviews, delineations of regions of interest, etc.). Further details of this would be provided further in the patent application. The obtained feedback could be transmitted to other nodes of the healthcare institution for obtaining further feedback from other participants of the healthcare network. As a final step, the clinical report is provided, which comprises highlighting at least one of: the regions of interest, the objects of interest, the clinical feedback from the at least one node in the healthcare network. The clinical report could be used for better healthcare delivery by the participants of the healthcare network. These details are given as an example only and are not limiting in any way. Further details would be provided further in the application.

In some embodiments, the method further comprises determination of regions of interest and/or objects of interest, which is based on a Machine Learning algorithm from at least one of: entity recognition algorithm, long-term short memory (LSTM), Conditional Random Field (CRF), Self-Attention. This list is non-exhaustive and other algorithms with similar functions can be envisioned.

The method could further comprise determination of regions of interest and/or objects of interest comprising the steps of: inputting the clinical information data and/or radiology examination data, extracting at least one named entity from the data, determining the relationships between named entities, mapping the named entities to a clinical guideline and/or historical patient data, outputting the result to an interpretability module and/or the diagnosis module.

The method could further comprise the step of determining the relationship among named entities includes the step of expert supervision and/or annotation. For example, the clinical specialist could have a possibility and/or means of annotating the radiology examination data. As a practical example, the clinical specialist could delineate the tumours for the subsequent therapy planning. For instance, one of the participants in the healthcare network is an oncologist trained to do the delineation, the oncologist delineates the tumour for subsequent radiation therapy planning, and sends it back to the referring physician indicating the delineated region for subsequent review of the referring physician.

In some embodiments, the method of clinical decision support further comprises an unsupervised transfer learning algorithm, such as a BERT (Bidirectional Encoder Representations from Transformers) model, is used for the step mapping of the named entities to a clinical guideline and/or historical patient data. Other algorithms with similar functions can be envisioned.

The method can be, in some instances, based on applying a down-convolution Neural Network on the raw data for the step of mapping of data. The step then further comprises performing the mapping on the raw data and applying an up-convolution Neural Network for receiving a fusion metric in a latent space, wherein the mapping comprises a feature map comprising named entities feature map and a relations feature map.

The method could further comprise a conditional random field (CRF) probability classifier model and/or the LSTM model that is applied to a fusion metric for extracting the context of the clinical information data. The clinical context could include any useful information for diagnosing the patient, for instance, if a patient has lung cancer and it a smoker, or if the patient has cardio-vascular diseases and is obese.

In some instance, the method further comprises steps, wherein the clinical information data is configured to be recorded and converted into text by a voice recognition device. The voice recognition device could be of any device type, such as a microphone.

In some instances, the method further comprises the step of highlighting in the clinical report, which highlights the regions or objects of interest, comprising any one of: quality measures, Region of Interest (ROI) detection, fracture detection, lesion detection, visualization of dependencies between the radiology examination data and the clinical information data. These examples are non-limiting. The main goal is to highlight the ROI's or OOI's to the participants of the healthcare network for an efficient review, and more efficient diagnosis.

In some instances, the method further comprises the step of adding additional information by a user to the clinical reports, which may comprise any one of: annotations, commenting, contouring. The provided examples are of non-limiting nature and other examples may be possible.

The method may further comprise steps of collecting feedback in the clinical report from at least one node of the healthcare network: radiologist node, physician node, technician node, pathology node, clinical laboratory node, patient input node. By nodes within the present application, information hubs are being implied. For instance, radiologist node could comprise any information hub to which radiologists have access and through which could provide clinical input. This could be DICOM stations, PACS, RIS, HIS, etc. The main goal here is to get radiologist's feedback. Analogously, patient input node could comprise any information from the patient through any means of communication, such as patient information system, teleradiology/telehealth system, and others.

A method of training of data for the method of clinical decision support is described. The method of training comprises training the radiology examination data by a convolutional neural network (CNN) model and/or training the clinical information data by an unsupervised machine learning model. In some embodiments, the method of training comprises steps, wherein the convolutional neural network module is trained for providing feedback to clinical nodes in the network.

A data processing system is further described, wherein the data processing system comprises a processor adapted to perform any of the steps of the clinical decision support method.

A computer program comprising instructions is described, which, when the program is executed by a computer, cause the computer to carry out any of the method steps of the clinical decision support method. According to an aspect of the present disclosure, a computer-readable medium that stores instructions is described.

The method, system and computer program/computer-readable medium can comprise one or more technical effects, which will be described further. The technical effects described are given merely as examples, and do not intend to limit the application in any way considering the 35 USC §101 or any other USC statutes.

As an example, the clinical decision support method and system described in the present application could facilitate efficient data exchange throughout different nodes of the clinical network.

As an example, the clinical decision support method and system described in the present application could facilitate more efficient clinical diagnosis by mapping the radiology examination data to clinical information data.

As an example, the clinical decision support method and system described in the present application could provide for an efficient means of highlighting clinically interesting objects and regions of interest with clinical context and/or physician references.

As an example, the clinical decision support method and system described in the present application could facilitate informed decision-making, by also acquiring feedback from and providing more context, to the patient.

As an example, the clinical decision support method and system described in the present application could help in providing more context and more information for a more informed decision-making to the clinical participants of the healthcare network, e.g., physicians.

As an example, the clinical decision support method and system described in the present application could provide for an improved means for training of junior clinical personnel by employing clinical context, annotations and other relevant information.

As an example, the clinical decision support method and system described in the present application could provide an improved response and recorded communication channel between clinicians and radiologists, avoiding the insufficient information transfer and unreliable reports.

As an example, the clinical decision support method and system described in the present application could provide for more explainable and interactive clinical reports.

As an example, the clinical decision support method and system described in the present application could provide more emphasis on the regions clinicians need most for clinical decision-making, thereby improving the efficiency of operations.

As an example, the clinical decision support method and system described in the present application could provide for a transparent and understandable medical decision-making environment, avoiding their confusion and anxiety.

Other advantages can be envisaged to the person of the ordinary skill in the art within the context of the present invention.

These and other aspects of the invention will be apparent from and elucidated with reference to the embodiment(s) described hereinafter.

### BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements. The following figures help in describing the invention:
Fig. 1 schematically depicts a clinical decision support method according to an embodiment of the present invention.
Fig. 2 schematically depicts mapping of data according to the present application.
Fig. 3 schematically depicts the clinical report obtained as a result of the mapping.
Fig. 4 schematically depicts a clinical workflow example in obtaining remote feedback by a radiologist.
Fig. 5 schematically depicts the clinical workflow including patient co-decision making.
Fig. 6 schematically depicts the diagnosis module.
Fig. 7 schematically depicts the interpretability module.
Fig. 8 schematically depicts an embodiment mapping that is being performed in the diagnosis module.
Fig. 9 schematically depicts an embodiment mapping that is being performed in the interpretability module.

### DETAILED DESCRIPTION OF EMBODIMENTS

The invention will be described with reference to the figures. The person of ordinary skill in the art would understand that further embodiments may be possible within the context of the present invention.

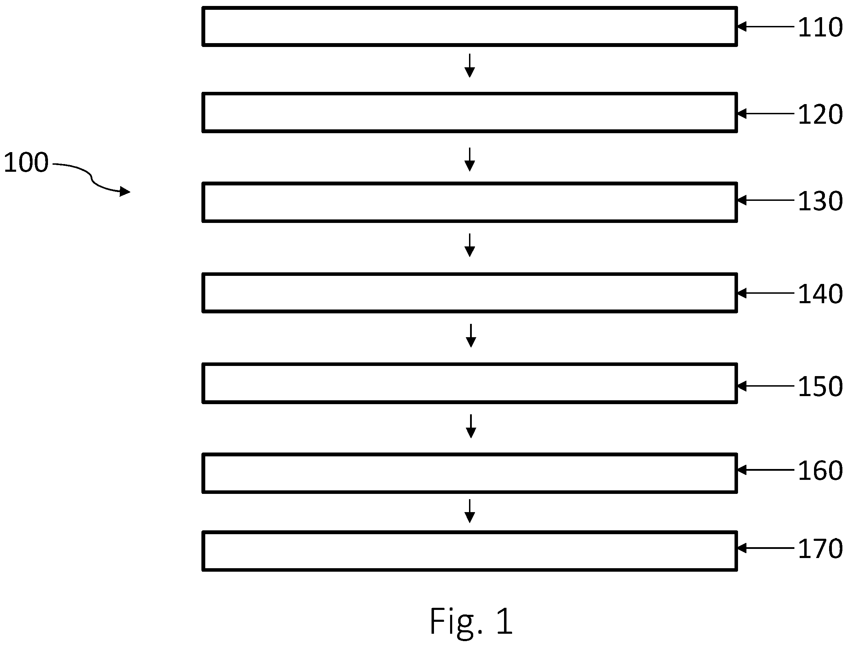

The detailed description and specific examples, while indicating exemplary embodiments of the apparatus, systems, and methods, are intended for purposes of illustration only and are not intended to limit the scope of the invention. These and other features, aspects, and advantages of the apparatus, systems and methods of the present invention will become apparent from the following description, appended claims, and accompanying drawings. The Figures 1-9 are merely schematic and are not drawn to scale. It should also be understood that the same reference numerals (e.g., 100, 200, 300) are used throughout the Figures to indicate the same or similar parts.

**Fig. 1** schematically depicts a clinical decision support method according to an embodiment of the present invention. In one aspect of the present invention, a method 100 for clinical decision support is described. The method could be a computer-implemented method. The method 100 comprises the steps of:
obtaining 110 radiology examination data 111 from at least one of the following modalities: imaging device, Digital Imaging and Communications in Medicine (DICOM) system, Picture Archiving and Communications (PACS) system;
obtaining 120 clinical information data 121 from at least one of the following modalities: electronic health record (EHR), electronic medical record (EMR), hospital information system (HIS), radiology information system (RIS), Systematized Nomenclature of Medicine-Clinical Terms (SNOMED-CT);
determining 130, by a diagnosis module 131, at least one object of interest 132 in the clinical information data 121, and determining 133, by an interpretability module 134, at least one region of interest 135 in the radiology examination data 111;
   mapping 140 the clinical information data 121 and/or the at least one object of interest 132 to the radiology examination data 131 and/or at least one region of interest 135 thereby obtaining a data mapping 141;
   transmitting 150 the clinical information data 121, the radiology examination data 131 and/or the data mapping 141 to at least one node in a healthcare network 142;
obtaining 160 clinical feedback 161 from the at least one node 162 in the healthcare network 142;
providing 170 a clinical report 171 comprising highlighting 172 at least one of: the regions of interest 135, the objects of interest 132, the clinical feedback 161 from the at least one node 162 in the healthcare network 142.

In some embodiments, the method (100) comprises means, such as computer system, for obtaining 110, 120 the radiology and clinical examination data. In some embodiments, the obtaining steps 110, 120 are done by a general-purpose computer or a dedicated-purpose computer, such as a specific processor. It is to be understood that the radiology examination data 111 could be of any type of data that could support a wide class of imaging devices: CT (Computed Tomography), MRI (Magnetic Resonance Imaging), US (Ultrasound), X-Ray, PET (Positron Emission Tomography), SPECT (Single-photon emission computed tomography), pathology, elastography, fluoroscopy, endoscopy, CBCT (cone-beam computed tomography) and any other device that is used within a clinical setting and produces an image as an end result. Variations of these devices are also envisioned within the current application, such as spectral CT. Preferably, the radiology examination data comprises at least one of: MRI images, CT images, US images, pathology images, or any combination of these images, like simulated CT/MR images. It is to be understood that these images can come directly from an imaging device, workstations like Digital Imaging and Communications in Medicine (DICOM) system, Picture Archiving and Communications (PACS) system, or any other related equipment that can obtain and/or store and/or transmit these images. The clinical information data 121 could be any clinical data, structured and/or unstructured, that contains any information about a specific patient/subject. The data could comprise any general information, such as age/sex, any lifestyle indications, such as smoker/non-smoker/obese/non-obese, and/or any clinical indications obtained from one or more diagnosis, like blood sample analysis, genetic/genomic tests, etc. that could help in understanding the context of the disease. This clinical information data 121 could come from any modality electronic health records (EHR), electronic medical record (EMR), hospital information system (HIS), radiology information system (RIS), Systematized Nomenclature of Medicine-Clinical Terms (SNOMED-CT), or any other modality that could comprise relevant patient data. This modality could be within a healthcare institution, or outside the healthcare institution, such as databases of insurance brokers/insurance providers.

The step of determining 130, by a diagnosis module 131, at least one object of interest 132 in the clinical information data 121, and determining 133, by an interpretability module 134, at least one region of interest 135 in the radiology examination data 111 is performed for analysing the information that could be of interest in the clinical decision-making. The interpretability module (134) and the diagnosis module 131 could be of any specific-purpose or general-purpose processors, such as GPU's (Graphical Processing Units), for determining regions of interest 135 in the radiology examination data 111, or CPU's (Central Processing Units), configured to determine objects of interest 132. It is to be understood that the objects of interest 132 and regions of interest 135 are collective terms. For instance, the objects of interest 132 could include words, keywords, phrases, sentences, numbers, graphs, tables, acronyms, visuals (e.g., blood visualizations), staining information, or any other information that could be derived from e.g., an EHR system. For instance, regions of interest 135 could include regions of interest, artifacts, lesions, contrast information, cine's, fractures, or any other information that could be derived, and is of interest, in a medical image.

The step of mapping 140 the clinical information 121 and/or objects of interest 132 to the radiology examination data 131 and/or at least one region of interest 135 for obtaining a data mapping 141 would be described more in-detail with respect to other embodiments. In some embodiments, this step includes mapping of one type of information (e.g., medical images) to other types of information (e.g., clinical reporting information) so that the physician has both the image information and the clinical information at hand for a fast review and/or improved speed of diagnosis. For instance, this could include determining lesions within a medical image, and determining factors that could contribute towards this diagnosis, so that the reviewing physician does not need to analyze all the information from the clinical reports, but can focus on a critical subset of information. As a practical example, consider a patient that has lung cancer, who is a smoker and had exposure to asbestos, which was determined from an intake evaluation. In this case, the system will determine on one hand that lung cancer may be present, and will also highlight the factors that have contributed to these, such as highlighting that the patient is/was a smoker and was exposed to asbestos. Furthermore, the system might determine objects of interest from clinical evaluations (e.g., biopsy results highlighting specific cancer stage). This will be factored together in a data mapping 141.

In some embodiments, the radiology examination data 131 could comprise multiple different types of medical images. Continuing the example of a smoker, CT images may be analysed for determining if cancer is present and highlighting regions of interest, whereas another set of images may include pathology imaging data for analysing the type of cancer (small cell lung VS non-small cell-lung). It is to be understood that the same stands also for clinical information data 121, wherein different types of data, such intake evaluation, blood samples, biopsy reports, could be analysed and mapped together.

Upon obtaining the data mapping 141, as well as the initial set of data comprising the radiology examination data 111 and the clinical information data 121 could be transmitted in step 150 to at least one node 162 in a healthcare network 142. The healthcare network 142 is a collective term, and could comprise institutions within the same hospital (e.g., a radiology department of the same hospital and an intensive care department) and beyond the same hospital (e.g., a radiology department of a different hospital, or an insurance company, or a clinical consulting site). The at least one node 162 generally represents a point in a network or diagram at which lines or pathways intersect or branch. The at least one node 162 represents stakeholders (e.g., clinicians from other specialities, patient) that would be interested in providing feedback on the mapping of data 141. Then, in step 160, the stakeholders could add additional information to the mapping of data (e.g., comments based on their knowledge or pose questions), provide further references on what needs to be done (e.g., referring based on the mapping that a further pathology study would be needed to confirm the findings), provide suggestions in the description of steps that have been performed, change the e.g. regions of interest, provide further highlights in the report that other stakeholders in the nodes should pay attention to, or otherwise add and/or alter the information that is provided in the report.

Finally, in step 170, the clinical report 171, which could comprise the highlighting (-s) 172 performed by the system and/or participants of the healthcare network 142 is transmitted and shown to at least one stakeholder in the healthcare network 142. It is to be understood that the highlights 172 are a general term, which comprises either stressing in some way the objects/regions of interest by the computer, for instance highlighting the lesions that have been identified, and/or comprising stressing in some way the objects/regions of interest by the user, for instance, where the user manually stresses a point of interest that should be reviewed by participants in the healthcare network 142. As a non-limiting examples, highlights 172 could comprise visually highlighting the information in a certain colour for other participants to focus. Other means of highlighting could be envisioned.

It is to be understood that the described here in relation to this embodiment, might also apply in other embodiments described further, and also is not intended to limit the application in any way. Other embodiments within the same general concept described here could be envisioned.

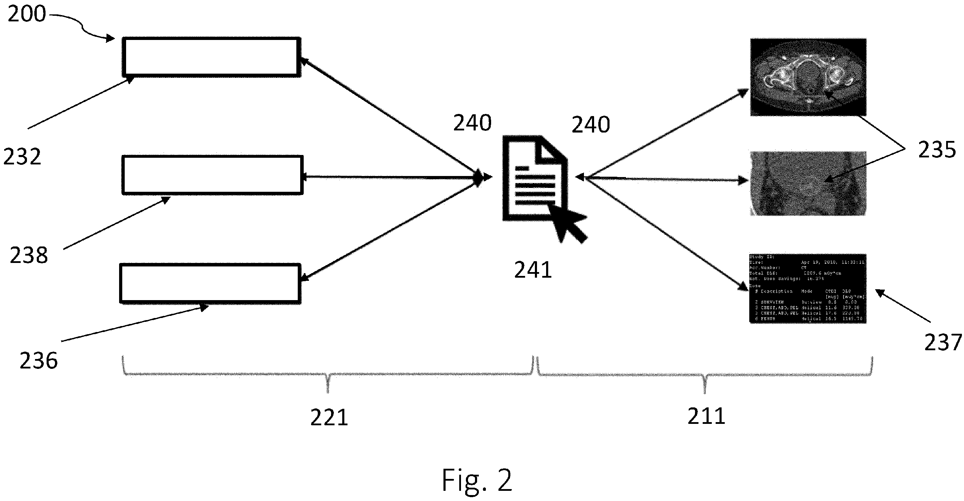

**Fig. 2** schematically depicts mapping of data according to the present application. According to this embodiment, the method 200 comprises obtaining clinical information data 221 and radiology examination data 211 and mapping this data to a data mapping 241.

According to this embodiment, the clinical information data 221 comprises of 3 parts: Machine Learning (ML) findings, wherein the machine learning extracts in step 232 the objects of interest from the clinical information data. This could include, for instance, keywords from an intake study, which are relevant in the clinical diagnosis.

Furthermore, the embodiment of fig. 2 includes radiologists' findings 238, for instance a detailed report that describes the results of an imaging study with radiologists' findings and suspected diagnosis. These findings could also be extracted with e.g., a machine learning algorithm from the clinical information system and could be transmitted in the further step of mapping.

Furthermore, the algorithm could further include determining 236 the links between the regions of interest 235 and the machine learning findings and radiologist findings 238 described in step a) and b) above. This further step could improve the precision of the mapping that will be done in subsequent steps.

According to this embodiment, the radiology examination data 211 comprises of 3 parts:
general radiology examination data 211 including the medical images
the regions of interests that have been determined either by the computer system and/or by the radiologists that have reviewed the images and have manually delineated the regions of interest and
clinical study information 237, which could include details on the clinical protocol used for receiving the radiology examination data, which could comprise further points of interest, such as the study protocol used, the regions examined, the dose received, etc.

In the step of mapping 240 all of the previously obtained information 211, 221, 232, 235, 236, 237 is mapped, wherein the mapping is done from the radiology examination 211 to clinical information data 221 and vice-versa. In doing so, the system can analyse the medical images from the radiology examination data 211 and associated regions of interest 235 in order to determine the possible nature of the disease, e.g., analysing the Hounsfield Units (HU's) and the images in order to determine possible suspicion of cancer. Then the system would consult the medical information data 221 in order to possibly identify additional factors that could prove such a diagnosis (e.g., radiologist conclusions, machine learning findings). If there is a possible match of the suspected diagnosis, the system will provide a data mapping 241 comprising both the radiology examination 211 and clinical information 221 data. If there is no match between the suspected findings, or the correlation is fairly weak, the system would repeat the procedure for other possible findings until there is a match between the clinical information data 221 and the radiology examination data 211. In other words, the algorithm would repeat itself until what the radiologists have concluded matches to the clinical images. If in all of the possible diagnosis, there is not any strong correlation between the clinical information data 211 and radiology examination data 221, the system would do the mapping 240 on the most probable diagnosis.

It is to be understood that the data mapping 241 could serve various benefits, such as improved co-decision making, quality assurance of the performed diagnosis to confirm the radiologists' findings, and others. It is to be further understood that various embodiments and alterations are possible, the example described in relation to embodiment of figure 2 is a non-limiting example.

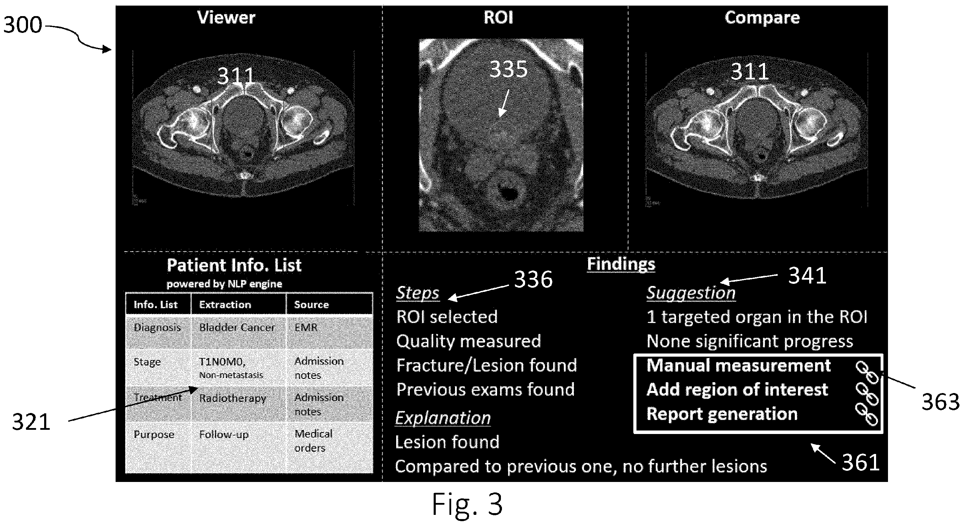

**Fig. 3** schematically depicts the clinical report obtained as a result of the mapping. The embodiment 300 shows an example of a clinical report 371 that is generated based on the mapped data 341 and that obtained the clinical feedback 361, which includes the radiology examination data 311, the at least one region of interest 335, the clinical information data 321. It is to be understood that the clinical report 371 provided in fig. 3 is a non-limiting example and other examples of clinical report might be possible within the scope of the present application. In this embodiment 330 the viewer shows the radiology examination data 311 on the left top corner, wherein the regions of interest 335 are shown on the top middle corner, and a comparison image, which could comprise both at least one medical image of the radiology examination data 311 with possible regions of interest 335 is displayed on the top-right corner. On the bottom left corner, the clinical information data 321 is displayed, which shows the information of interest (diagnosis, proposed treatment, etc.), the clinical conclusion (e.g., bladder cancer and radiotherapy cancer), and the source of the clinical information (e.g., EMR and admission notes). On the central portion of the image, the determined links 236 between the regions of interest, the data mapping 241 (it is to be understood that this is not mapping per se that is done in the background, but merely reflecting some results of the mapping), and the clinical feedback 361 that was obtained from at least one participants of the network. As was previously described, the clinical feedback could be exhibited in various forms, and this is just one non-limiting example of the clinical feedback. It is to be understood that by providing the clinical feedback 361, the referring stakeholder can also assign tasks 363 to other participants of the network, wherein the stakeholders will add the additional information according to the assigned tasks 363.

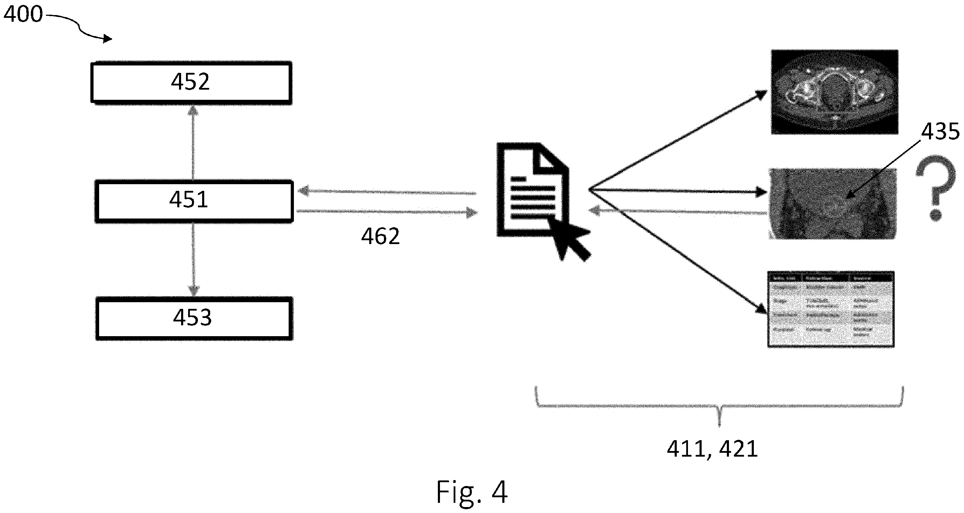

**Fig. 4** schematically depicts a clinical workflow example. In the example of this embodiment, there is a suspicion of a lesion that is being detected by a referring physician 451, and wherein the region of interest 435 is being determined by the referring physician, by the machine learning algorithm, the by the computer system, or by any other application/medical specialist. In this case the referring physician 451, who is not certain that it is a lesion and not e.g., an artifact, refers the potential finding with a region of interest 435 to a radiologist 452. The radiologist analyses the radiology examination data 411, including the region of interest 435, which represents the suspected finding, and e.g., confirms the finding and transmits this information back to the referring physician 451, and provides the clinical feedback 462 to the data mapping 441. However, since cancer determination is a complex endeavour, a further confirmation from a pathologist 453 is needed. Referring physician transmits a further reference to the pathologist 453, wherein the pathologist 453 analyses the radiology examination data 411, clinical information data 421, the clinical feedback 462 from the radiologist 452, the data mapping 441, and provides either confirms the finding or does not confirm the finding. The referring physician then provides the clinical feedback 462. If all 3 clinical specialists 451, 452, 453 confirm the findings in this example, then the patient probably has cancerous tissue and needs to be treated by e.g., radiation therapy. Generally, this embodiment shows how the system that has been described previously in any of the previous embodiments can be used for clinical co-decision making. This is a representative example, other embodiments with the same context may be envisioned.

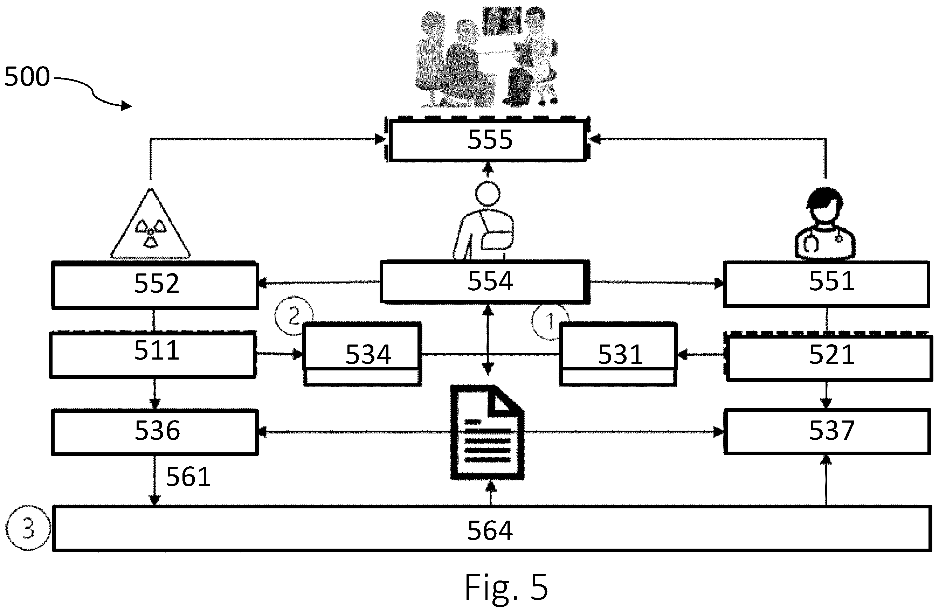

**Fig. 5** schematically depicts the clinical workflow including patient co-decision making. In the present embodiment the radiology examination data 511 and the clinical information data 521 is obtained. The objects of interest are determined by the interpretability module 531 and the regions of interest are determined by the diagnosis module 534. This information is then transmitted to the referring physician 551 and the radiologist 552, who have access to this information for further analysis, including determination of the links between the regions of interest 236 and the clinical study information 237. A data mapping 551 can be performed and clinical feedback 561 by various specialists is provided. The clinical feedback can be provided through an interactive module 564. In some embodiments, the interactive module is a Graphical User Interface (GUI). The clinical feedback can be transmitted to stakeholders of the healthcare network 542 and/or to the data mapping 551 and/or provide to the clinical report 571. It is to be understood that the patient 554 has access to different nodes in the healthcare network 542, such as the radiologist 552, the referring physician 551, the clinical report 571. The patient 554 has the possibility of providing comments/questions/requests for clarification to any of the participant of the nodes of healthcare network 542 in any stage of the clinical care. This would allow to achieve a more efficient co-decision making 555. It is to be understood that this embodiment is exemplary, and other variations to the clinical workflow is possible.

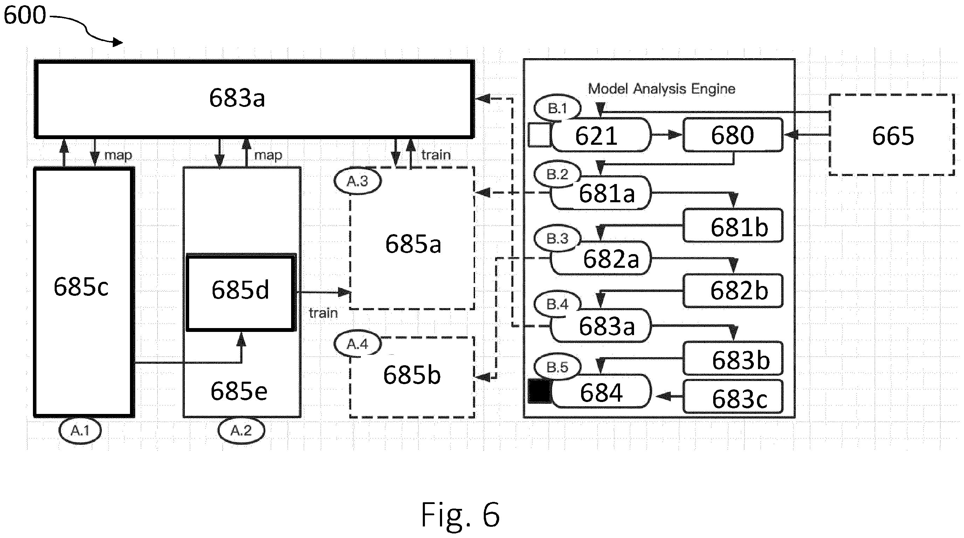

**Fig. 6** schematically depicts the diagnosis module 634. According to this embodiment, clinical information data 621 is being input at step B 1, which can be extracted from e.g., EHR, or inputted from a voice recognition device 665 from the participants of the healthcare network 642. The clinical information data 621 being analysed by a diagnosis model 680. The diagnosis model 680 could be of any model type that is configured to analyse structured or unstructured medical data, such as models based on statistical/stochastic algorithms, models based on machine learning algorithms, and others. In the preferred embodiment, the diagnosis model 680 is based on a machine learning algorithm, such as an entity recognition algorithm. At step 681a, named entities are extracted. Named entities could be, for example, words/phrases that have similar attributes, such as comprising numbers (numerical attributes), or comprising some medical diagnosis. Based on the extracted named entities 681a, a further extraction of potential concepts can be performed in step 682b. The potential concepts can include a pre-selection of possible medical diagnosis applicable to a certain patient based on the clinical information data 621 and the extracted named entities 681a. In some embodiments, machine learning approaches, like supervised and/or unsupervised learning can be used to enhance the extraction of the models. At the next step, a quality assurance module 682 is applied to screen for potentially incorrect potential concepts. The quality assurance module 682 is also configured to supervise the potential concepts within the clinical context, such as determining the clinical the clinical context for the determined potential concepts, and if the clinical context fits the potential concepts. If the clinical context does not fit the potential concepts, this might represent an incorrect diagnosis and could be filtered out by the quality assurance module 682a. At step 682b, the key clinical facts are being determined. The key clinical facts represent the most promising potential diagnosis and/or the most essential information for further clinical study.

At step 683a a knowledge mapping is performed for extracting the key information 683b. In some embodiments, the key information 683b may mean the key features 682b comprising a step of knowledge mapping. In some embodiments, the key features 682b would be mapped to the key information 683b through an additional step of knowledge mapping using SNOMED-CT or other medical knowledge system. A non-limiting example may comprise be as follows. Key feature 682b: patient has been diagnosed with a brain tumour, key information 683b: Location of the tumour in a medical image. SNOMED CT code that is used for knowledge mapping: 126906006 (Brain tumour). Based on this information, a mapping of the key feature "Patient has been diagnosed with a brain tumour" to the key information "location of tumour" is performed by e.g., first identifying the SNOMED CT code for brain tumour using named entity recognition. The code is used to retrieve information about the type and location of tumour, and current treatment plan from a database or electronic health record system. This information can be used to inform treatment decisions.

In some embodiments, the system further extracts historical data 683c that could give more context around the key information 683b, such as the lifestyle of the patient and/or previous diseases. The historical data could include any one of: previous examination, key information from the medical diagnosis, and other information comprising patient history. At step 684 the key information 683b and the historical data 683c could be combined and analysed by a machine learning algorithm to provide clinical suggestions. To enhance the clinical process, the diagnosis module 634 could also utilize voice recognition from the voice recognition device 665 during the process to capture the key information from patients' end as well, e.g., patients' recollection of key historical data that could aid further diagnosis. Voice recognition allows clinicians to relay a patient's care narrative verbally, and have it transcribed into e.g., an Electronic Health Record (EHR). This could provide for the key information from both clinicians and patients, and can be used by radiologists or other clinicians later in the clinical care process.

In some embodiments, further steps of training and mapping could be used for improving the clinical diagnosis. The diagnosis module 634 could be configured for training 685a of named entities, as well as context analysis and further quality check 685b by the participants of the healthcare network 642. This would allow to increase the precision of the diagnosis module 634 and decrease the instances of incorrect diagnosis and/or incorrect context. In some embodiments, domain knowledge could be analysed to extract the main relevant clinical concepts from a domain knowledge database 685c, the extracted information could be further mapped to knowledge mapping module 683a and/or transmitted to a domain corpus of medical notes 685d, wherein a processor module 685e can perform further text processing and/or map to the knowledge mapping module 683a. The result could be transmitted to the knowledge mapping module 683a.

In some embodiments, expert supervision and annotation can be used to extract entity and relationships among named entities. A transfer learning model as a first encoding module can be used. For instance, BERT model can be used for embedding the entities and relations into feature map respectively. Furthermore, convolution to down sampling can be done, which will downsample the convolution into higher dimension. In this case, the knowledge mapping (683a) and/or data mapping 641 is done in a high-level latent space. In some embodiments, a transpose convolution is used to up sample all these features into the original dimension, fuse together into the latent space the residual connection in the original dimension. In some embodiments, a CRF probability classifier and/or LSTM (Long Short-Term Memory network) can be used for improving the accuracy and rationality of named entity recognitions by taking into account the context and patterns in the data.

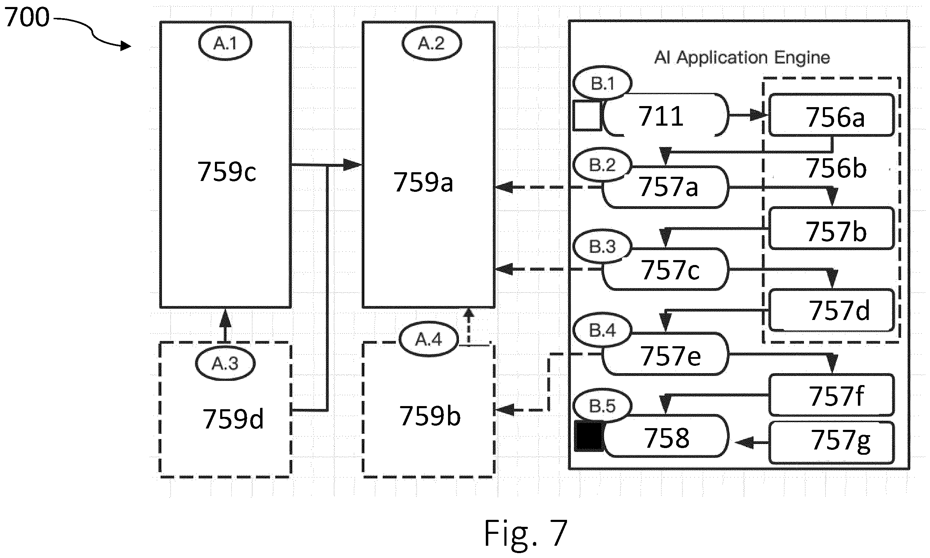

**Fig. 7** schematically depicts the interpretability module 734. The workflow has similarities to the diagnosis module in terms of steps, but this workflow of events is tailored towards medical examination data 711. Radiology examination data 711 is being input at step B1, which can be extracted from e.g., an imaging device, or inputted from e.g., a PACS database, which is transmitted to a participant of the healthcare network at step 756a. In some embodiments, the interpretability module is based on a machine learning algorithm, preferably an AI application engine.

In some further embodiments, an AI coordinator 756b is used. The AI coordinator could have means, such as a specific purpose processor, for coordinating 757a the data to an AI application 757b at step B.2 in fig. 7. In some embodiments, the data is transmitted 757c to a post-processing engine 757d, wherein the AI coordinator utilizing the post-processing engine would be able to transform the model output to the uniform demonstration integrated onto the interactive display at B.3 with the help of models of A.2. It would appreciated that various AI applications managed by the AI coordinator will have different format of outputs, such as bounding box, mask or classification. In order to integrate this information in an interactive display, a post-processing engine can be configured to provide for such configuration using some pre-defined formats and/or demonstration rules. It would be further appreciated that when using other AI applications from different vendors, the post-processing engine can be configured to uniform the output of the models. This step ensures the consistency of demonstration rules and easy for users to learn. Thereby, processed radiology examination data is obtained. Regions of interest 735 could be also extracted at this step.

At optional step B.4, a linking 757e of the processed radiology examination data, including the regions of interest 735 is performed, wherein the result of linking 757f is outputted as processed radiology examination data, including regions of interest 735. In some embodiments, further text from the report (e.g., dose information, exmination information) is being extracted and added to the result of the linking. Futher, historical data 757g could be inputted as an additional data point for better determination of regions of interest. For instance, historical data could be atlas data or past patient radiology examination, that could be used for comparison with the radiology examination data 711 and the processed radiology examination data. At step 758 radiology suggestions can be generated taking into account radiology examination data 711, regions of interest 735, historical data 757g and/or the results of the linking 757f. In some emboduments, the radiology suggestions 758 could be of any one of: report, natural language with findings, radilogical images.

In some embodiments, task models 759a could be further used for improving the precision of the AI application engine and/or the interpretability module 734. To make the task model 759a, which could be based on convolutional neural network models, more powerful, a versatile model structure to fulfill the main computer vision tasks, such as classification/detection/segmentation could be deployed. In other words, the task model 759a could be configured for performing classification, detection and segmentation.

In some embodiments, the task model 759a comprises a transfer learning model based on ImageNet or other medical image big databases that is configured to capture the basic textures and features from the radiology examination data 711. A uniform task prompt performed by a prompt encoder 759b, which is configured for lesion classifiation/detection/segmentation or quality classification/detection/segmentation could be further used. In some embodumetns, the prompt encoder is configured to perform specific target imaging. In some embodiments, the specific target imaging comprises any one of labelling, masking, bounding the box, and other operators used for medical imaging processing.

In some embodiments, a feature map is embedded into the processed radiology examination data. The feature map could be down-sampled into high-level dimension, the prompt embedding from prompt encoder would be fused together. In the end, a processed radiology examination data, and radiological task created as a result, would be combined, and transposed convoluted into the original image spacing. In some embodiments, image processing models 759c could be further used for increasing the quality of the interpretability module. The image processing models 759c could be based on deep learning models configured to be used for image processing and/or computer vision applications. In some embodiments, a link to 3^{rd} party vendors 759d is established, wherein the link 759d could provide with additional processing power and/or information that could improve the operations of the interpretability module 734.

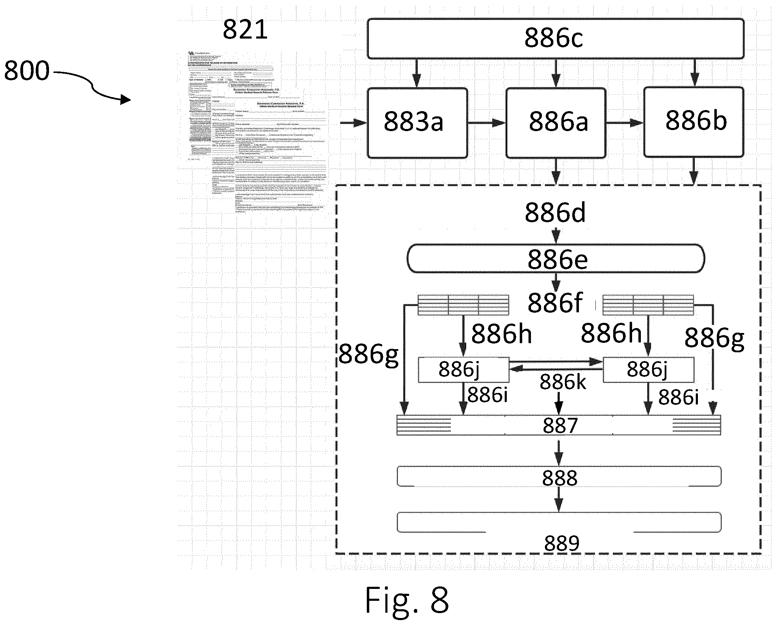

**Fig. 8** schematically depicts the mapping that is done in the diagnosis module 831. The clinical information data 811 is being inputted to the diagnosis module 834. In some embodiments, the input of clinical information data is being inserted in the knowledge mapping module 883a. This is transmitted to an entity recognition module 886a, which recognizes the main entities in the knowledge mapping module 883a. This could be transmitted to relationship module 886b, which could extract the main relationship between the data. It is to be understood, that expert supervision through the expert supervision module 886c could be done on 883a, 886a, 886b, and medical information data result could be generated that is translated to a natural language models 883d configured for medical information data analysis. At the next step 886e unsupervised pre-trained models are applied. At step 886f, entity feature maps are being generated.

The entity feature maps can performed onto 2 classes of data, wherein on the first class and on the second class of data, a residual connection analysis 886g is performed. In some embodiments, a pre-trained model, such as BERT, is used. The pre-trained model can be configured for named entity and relation extraction. In some embodiments these may be done as separate non-concurrent steps, in some embodiments as concurrent steps. As a result, two feature maps, namely entity feature map and relation feature map, can be generated. This result is used as a first input for a fusion metric 887. In some embodiments, a residual connection is implemented for two feature maps respectively. The focus of the residual connection is to restore the information for two feature maps and fuse them in a latent space at the original dimension.

Furthermore, on the first and the second class of data, down-convolution 886h is performed, wherein high-dimensionality data is obtained 886j, wherein a step of mapping of high dimensionality data 886k could be performed. It is to be appreciated that in the domain of natural language processing (NLP), high-dimensionality data can refer to abstract representations of text that captures complex semantic and syntactic patterns in some language. These features can often learned by the machine learning algorithm through a process called feature extraction. In some embodiments, a pre-trained BERT and down-convolution of a Convolutional Neural Network (CNN) is performed for obtaining higher level features. In some examples, the output is a multi-dimension feature matrix. It would be further appreciated that the step 886k can be used to fuse the high-level dimensionality data of two tasks in the higher dimension latent space. This is one of the advantages of the current approach in order to ensure the matching between entities and relations.

In the next step, up-convolution (886i) is performed. The result is used a second input for a fusion metric. From the fusion metric, query from domain knowledge database is being performed, whereas a probability classifier is being applied at step 888. The probability classifier is preferably an CRF and/or LSTM model. As a result, potential concepts 889 are being derived, which could be applied to the embodiment of figure 6, or used as a separate embodiment. In some embodiments, the mapped data can be combined, wherein the two separate feature maps are also up sampled to be fused.

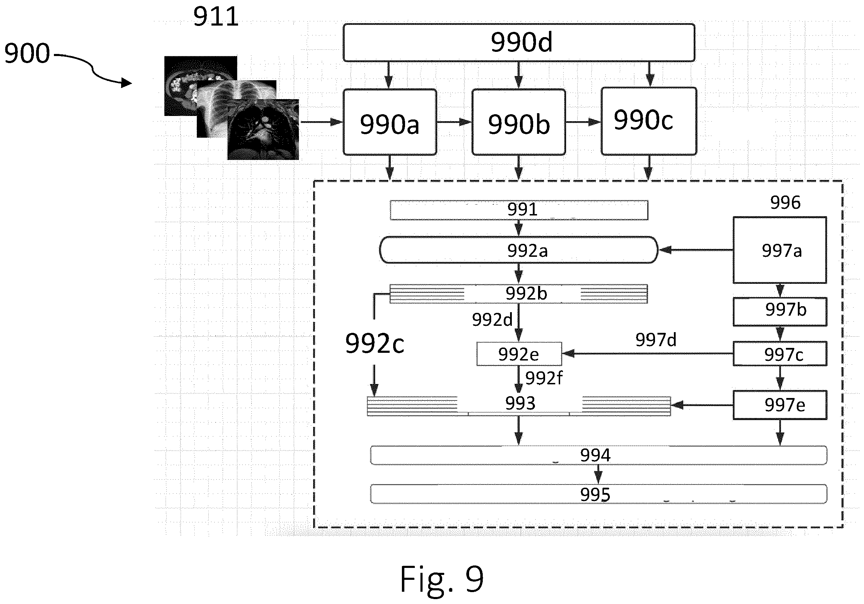

**Fig. 9** schematically depicts an embodiment mapping that is being performed in the interpretability module 934. Radiology examination data 911 is being inputted into a classification module 990a, which is configured for classifying the medical images into certain categories. As a non-limiting example, the images could be classified per modality type, such as CT and MR images. The detection module 990b detects the potential findings within images, such as potential lesions. The segmentation module 990c is then configured for segmenting of images, so that the potential findings detected by the detection module could be more easily processed and/or represented during future steps. It is to be understood, that any of the modules 990a, 990b, 990c could be connected to expert supervision and annotation module 990d, wherein a clinical specialist, such as a radiologist, can provide supervision and annotation, such as image delineation. The result obtained from any of the modules 990a, 990b, 990c is transmitted to an imaging module 991, wherein to the imaging module a transfer learning model is applied 992a, wherein a feature map 992b is generated as a result. A residual connection 992c is applied that serves as a first input into a fusion metric 993. Furthermore, a down-convolution network 992d can be applied to the fusion metric, which results in high-dimensionality data 992e, wherein an up-convolution network 992f is applied for generating a second input to the fusion metric 993 similarly to the embodiment of fig. 8. The result of the combined input of the fusion metric 993 is transmitted to an image module 994, which results in a final mask 995 in the original image space.

In some embodiments, a task module is being used 996 configured for providing further task input to the interpretability module 934. The task module 996 is configured to highlight, or otherwise stress, the regions of interest 935, by detecting the regions of interest 997a, such as lesions, fractions, nodules, etc. The prompt encoder 997b encodes the regions of interest and transmits the result to the task dimensionality module 997c. This allows to train the model to be versatile enough to perform the different computer vision tasks. For example, for classification, the model can be trained to recognize different object categories, for detection, train the model to detect and localize objects, and for segmentation, training the model to segment the image into different regions based on object categories.

In some embodiments, the task dimensionality module 997c is configured to fuse 997d the data with the high-dimensionality data 992e. The result of the task dimensionality module 997c is transmitted to a task decoder 997e. The task decoder 997e is configured to transmit the output to the fusion metric as a third input and/or directly to the image module 994. In some examples the input would be a prompt in natural language, and the output could be an e.g., segmentation. This is a representative example, and other examples may be possible within the context of the present invention.

It is to be appreciated that this embodiment may have an architecture of an encoder-decoder type, such as the one described in "Understanding How Encoder-Decoder

Architectures Attend" by K.Aitken et.al., which is hereby incorporated by reference, and can be applicable to any of the embodiments described herein. The encoder-decoder architecture comprises of two main components: the encoder and the decoder. The encoder is responsible for transforming the input data into a compressed representation, also known as the "latent space". The decoder, on the other hand, takes the compressed representation generated by the encoder and transforms it back into the original form.

In some embodiments, the data that is used as input for the diagnosis module of any of the previous embodiments, can be further trained. The training comprises the steps of:
Pre-processing: pre-processing the clinical information data data in a natural language by tokenizing, stemming or lemmatizing, removing stop words, and /or converting the text to numerical representations first if necessary. As a result, pre-processing output is obtained.

Pre-training the pre-processing output using BERT model, or similar models. BERT is a powerful pre-trained model that has achieved state-of-the-art results on many NLP tasks. As a result, pre-training model data is obtained.

Obtaining the pre-trained model data and fine-tuning the data on a specific domain data for a specific task, such as named entity recognition, relationship extraction and others that were described in any of the previous embodiments. The fine-tuning process involves further training the pre-trained BERT model on the task-specific dataset. The model is initialized with the pre-trained weights from the BERT model and is then further trained on the task-specific dataset using backpropagation to adjust the weights of the model. This may involve adding a new layer on top of the pre-trained BERT model and training the model using supervised learning on the labeled dataset. The BERT model is trained as a feature extractor, and a new layer is trained to make predictions on our specific domain such as radiology reports. Metrics are obtained as a result.

Fusing the metrics in latent space. After embedding from pre-trained model for the entity and relation feature map, a down-convolution is used to acquire the features and fuse them in the higher dimensions. Then the residual connection and up-convolution could be trained to fuse them in the original dimension.

Applying LSTM/CRF layers. In addition to the pre-trained BERT and fusion, in some instances LSTM/CRF layers can be added to the system to further improve its performance on NLP tasks. The LSTM layers can be trained to capture long-term dependencies in the text, while the CRF layer can help improve the accuracy of sequence labeling tasks like named entity recognition.

Using data augmentation. Data augmentation techniques such as random word replacement, synonym replacement, and back-translation can be used to generate new training data and improve the robustness of the model.

Using ensembled learning. Ensemble learning can also be used to combine the predictions of multiple models, including pre-trained models like BERT and other CNN models, to improve the accuracy of the final result.

In some embodiments, the data that is used as input for the interpretability module of any of the previous embodiments can be trained. The training comprises the steps of:
Defining the task module inputs. For instance, defining a set of task prompts for different computer vision tasks. For instance, for classification, the prompt could be "classify the object in the image", for detection, the prompt could be "detect the object(s) in the image and localize them", and for segmentation, the prompt could be "segment the image into different regions based on object categories".

Collecting and labelling the data. Collect a dataset of images and labelling them according to the different task prompts. For example, for classification, label the images with the object category, for detection, label the images with the object category and the bounding box coordinates, and for segmentation, label the images with the object category and the segmentation mask.

Pre-processing the data: Pre-process the image data by resizing, cropping, normalizing, and augmenting the images to improve the model's performance and reduce overfitting.

Choosing a pre-trained model. Choosing a pre-trained model as a starting point for the training process. For example, using a pre-trained ResNet model for classification, a pre-trained Faster R-CNN model for detection, and a pre-trained Mask R-CNN model for segmentation.

Transfer learning for fine-tuning: Fine-tune the pre-trained model on our labeled dataset using transfer learning techniques. Use the task prompts to train the model to perform various computer vision tasks. For example, for classification, training the model to recognize different object categories, for detection, train the model to detect and localize objects, and for segmentation, train the model to segment the image into different regions based on object categories. As a result, metrics are obtained.

Fusing the obtained metric in latent space: After embedding from pre-trained model for the medical imaging feature map, applying a down-convolution model to acquire the features and fuse them with task features in the high dimension of the latent space. Furthermore, residually connecting, and applying an up-convolution model, that could be trained to fuse them in the original dimension with the prompt task decoder.

Post-processing the results. With the corresponding results generated by the task prompt, the post processing would transform image and task results into the original image space and make the visualization.

In some embodiments, the training is done for an NLP model.

It is to be further understood that transfer learning for fine-tuning is a deep learning technique that involves using a pre-trained model as a starting point for a new task and fine-tuning its parameters on a smaller dataset specific to the new task. By fine-tuning the pre-trained model on a smaller dataset specific to the new task, the model can adapt it to a new task and improve its performance.

It is to be understood that any of the previous steps described in relation to embodiments and/or training steps described above can be performed by a specific-purpose computer system or general-purpose computer system, or a computer-readable medium, data carrier system, that is configured to carry out any of the steps described previously. The computer system can include a set of software instructions that can be executed to cause the computer system to perform any of the methods or computer-based functions disclosed herein. The computer system may operate as a standalone device or may be connected, for example, using a network, to other computer systems or peripheral devices. In embodiments, a computer system performs logical processing based on digital signals received via an analogue-to-digital converter.

In a networked deployment, the computer system operates in the capacity of a server or as a client user computer in a server-client user network environment, or as a peer computer system in a peer-to-peer (or distributed) network environment. The computer system can also be implemented as or incorporated into various devices, such as a server or another type of computer such as a workstation that includes a controller, a stationary computer, a mobile computer, a personal computer (PC), a laptop computer, a tablet computer, or any other machine capable of executing a set of software instructions (sequential or otherwise) that specify actions to be taken by that machine. The computer system can be incorporated as or in a device that in turn is in an integrated system that includes additional devices. In an embodiment, the computer system can be implemented using electronic devices that provide voice, video or data communication. Further, while the computer system is illustrated in the singular, the term "system" shall also be taken to include any collection of systems or sub-systems that individually or jointly execute a set, or multiple sets, of software instructions to perform one or more computer functions.

The computer system may also include a processor. The processor executes instructions to implement some or all aspects of methods and processes described herein. The processor is tangible and non-transitory. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The processor is an article of manufacture and/or a machine component. The processor is configured to execute software instructions to perform functions as described in the various embodiments herein. The processor may be a general-purpose processor or may be part of an application specific integrated circuit (ASIC). The processor may also be a microprocessor, a microcomputer, a processor chip, a controller, a microcontroller, a digital signal processor (DSP), a state machine, or a programmable logic device. The processor may also be a logical circuit, including a programmable gate array (PGA), such as a field programmable gate array (FPGA), or another type of circuit that includes discrete gate and/or transistor logic. The processor may be a central processing unit (CPU), a graphics processing unit (GPU), or both. Additionally, any processor described herein may include multiple processors, parallel processors, or both. Multiple processors may be included in, or coupled to, a single device or multiple devices.

The term "processor" as used herein encompasses an electronic component able to execute a program or machine executable instruction. References to a computing device comprising "a processor" should be interpreted to include more than one processor or processing core, as in a multi-core processor. A processor may also refer to a collection of processors within a single computer system or distributed among multiple computer systems. The term computing device should also be interpreted to include a collection or network of computing devices each including a processor or processors. Programs have software instructions performed by one or multiple processors that may be within the same computing device or which may be distributed across multiple computing devices.

The computer system further includes a main memory and a static memory, where memories in the computer system communicate with each other and the processor via a bus. Either or both of the main memory and the static memory may be considered representative examples of the memory of the controller, and store instructions used to implement some or all aspects of methods and processes described herein. Memories described herein are tangible storage mediums for storing data and executable software instructions and are non-transitory during the time software instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The main memory and the static memory are articles of manufacture and/or machine components. The main memory and the static memory are computer-readable mediums from which data and executable software instructions can be read by a computer (e.g., the processor). Each of the main memory and the static memory may be implemented as one or more of random access memory (RAM), read only memory (ROM), flash memory, electrically programmable read only memory (EPROM), electrically erasable programmable read-only memory (EEPROM), registers, a hard disk, a removable disk, tape, compact disk read only memory (CD-ROM), digital versatile disk (DVD), floppy disk, Blu-ray disk, or any other form of storage medium known in the art. The memories may be volatile or non-volatile, secure and/or encrypted, unsecure and/or unencrypted.

"Memory" is an example of a computer-readable storage medium. Computer memory is any memory which is directly accessible to a processor. Examples of computer memory include, but are not limited to RAM memory, registers, and register files. References to "computer memory" or "memory" should be interpreted as possibly being multiple memories. The memory may for instance be multiple memories within the same computer system. The memory may also be multiple memories distributed amongst multiple computer systems or computing devices.

As shown, the computer system further includes a video display unit, such as a liquid crystal display (LCD), an organic light emitting diode (OLED), a flat panel display, a solid-state display, or a cathode ray tube (CRT), for example. Additionally, the computer system includes an input device, such as a keyboard/virtual keyboard or touch-sensitive input screen or speech input with speech recognition, and a cursor control device, such as a mouse or touch-sensitive input screen or pad. The computer system also optionally includes a disk drive unit, a signal generation device, such as a speaker or remote control, and/or a network interface device.

In an embodiment, the disk drive unit includes a computer-readable medium in which one or more sets of software instructions are embedded. The sets of software instructions are read from the computer-readable medium to be executed by the processor. Further, the software instructions, when executed by the processor, perform one or more steps of the methods and processes as described herein. In an embodiment, the software instructions reside all or in part within the main memory, the static memory and/or the processor during execution by the computer system. Further, the computer-readable medium may include software instructions or receive and execute software instructions responsive to a propagated signal, so that a device connected to a network communicates voice, video or data over the network. The software instructions may be transmitted or received over the network via the network interface device.

In an embodiment, dedicated hardware implementations, such as application-specific integrated circuits (ASICs), field programmable gate arrays (FPGAs), programmable logic arrays and other hardware components, are constructed to implement one or more of the methods described herein. One or more embodiments described herein may implement functions using two or more specific interconnected hardware modules or devices with related control and data signals that can be communicated between and through the modules. Accordingly, the present disclosure encompasses software, firmware, and hardware implementations. Nothing in the present application should be interpreted as being implemented or implementable solely with software and not hardware such as a tangible non-transitory processor and/or memory.

In accordance with various embodiments of the present disclosure, the methods described herein may be implemented using a hardware computer system that executes software programs. Further, in an exemplary, non-limited embodiment, implementations can include distributed processing, component/object distributed processing, and parallel processing. Virtual computer system processing may implement one or more of the methods or functionalities as described herein, and a processor described herein may be used to support a virtual processing environment.

The illustrations of the embodiments described herein are intended to provide a general understanding of the structure of the various embodiments. The illustrations are not intended to serve as a complete description of all of the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

One or more embodiments of the disclosure may be referred to herein, individually and/or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

The preceding description of the disclosed embodiments is provided to enable any person skilled in the art to practice the concepts described in the present disclosure. As such, the above disclosed subject matter is to be considered illustrative, and not restrictive, and the appended claims are intended to cover all such modifications, enhancements, and other embodiments which fall within the true scope of the present disclosure. Thus, to the maximum extent allowed by law, the scope of the present disclosure is to be determined by the broadest permissible interpretation of the following claims and their equivalents and shall not be restricted or limited by the foregoing detailed description.

### Reference Numerals:

100: method for clinical decision support
110: step of obtaining radiology examination data
111: radiology examination data
120: step of obtaining clinical information data
121: clinical information data
130: step of determining at least one object of interest
131: diagnosis module
132: object of interest
133: step of determining at least one region of interest
134: interpretability module
135: region of interest
236: determining the links between the regions of interest
237: clinical study information
238: radiologists findings
140: the step of mapping the clinical information, OOI to radiology examination data, ROI
141: the step of data mapping/data mapping
142: healthcare network
150: the step of transmitting the information
451: referring physician
452: radiologist
453: pathologist
554: patient
555: step of co-decision making
756a: transmittal of data
756b: AI coordinator
757a: transmittal of data
757b: AI coordinator
757c: data transmission
757d: post-processing engine
757e: linking of the processed radiology examination data
757f: result of linking
757g: historical data
758: radiology suggestions
759a: task model
759b: prompt encoder
759c: image processing models
759d: link to 3^{rd} party vendors
160: the step of obtaining clinical feedback
161: clinical feedback
162: at least one node in a healthcare network
363: the step of assigning tasks/assigned tasks
564: interactive module
665: voice recognition device
170: the step of providing a clinical report
171: clinical report
172: highlighting regions of interest, objects of interest, clinical feedback
680: diagnosis model
681a: step of extraction of named entities.
681b: step of extraction of potential concepts
682a: quality assurance module
682b: extraction of key clinical facts
683a: knowledge mapping
683b: key information
683c: historical data
684: clinical suggestions
685a: training of the named entities
685b: context analysis and further quality check by participants of the healthcare network
685c: domain knowledge database
685d: domain corpus of medical notes
685e: processor module
883a: knowledge mapping module
886a: entity recognition module
886b: relationship module
886c: expert supervision module
886d: natural language models for medical information data analysis
886e: unsupervised pre-trained models
886f: entities feature maps
886g: residual connection analysis
886h: down-convolution
886j: high-dimensionality data
886k: mapping of high dimensionality data
886i: up-convolution
887: fusion metric
888: probability classifier
889: potential concepts
990a: classification module
990b: detection module
990c: segmentation module
990d: expert supervision and annotation module
991: imaging module
992a: transfer learning module
992b: feature map
992c: residual connection
992d: down-convolution network
992e: high-dimensionality data
992f: up-convolution network
993: fusion metric
994: image module
995: final mask
996: task module
997a: detecting regions of interest
997b: prompt encoder
997c: task dimensionality module
997d: fusion
997e: task decoder

## Claims

1. A method for clinical decision support, the method comprising the steps of:
obtaining radiology examination data from at least one of the following modalities: imaging device, Digital Imaging and Communications in Medicine, DICOM, system, Picture Archiving and Communications, PACS, system;
obtaining clinical information data from at least one of the following modalities: electronic health record, EHR, electronic medical record, EMR, hospital information system, HIS, radiology information system, RIS, Systematized Nomenclature of Medicine-Clinical Terms, SNOMED-CT;
determining, by a diagnosis module, at least one object of interest in the clinical information data, and determining, by an interpretability module, at least one region of interest in the radiology examination data;
mapping the clinical information data and/or the at least one object of interest to the radiology examination data and/or at least one region of interest thereby obtaining a data mapping; transmitting the clinical information data, the radiology examination data and/or the data mapping to at least one node in a healthcare network;
obtaining a clinical feedback from the at least one node in the healthcare network;
providing a clinical report comprising highlighting at least one of: the regions of interest, the objects of interest, the clinical feedback from the at least one node in the healthcare network.

2. The method according to claim 1, wherein the determination of regions of interest and/or objects of interest is based on a Machine Learning algorithm in particular: entity recognition algorithm, long-term short memory, LSTM, Conditional Random Field, CRF, Self-Attention.

3. The method according to any one of claims 1 or 2, wherein the determination of regions of interest and/or objects of interest comprises: inputting the clinical information data and/or radiology examination data, extracting at least one named entity from the data, determining the relationships between named entities, mapping the named entities to a clinical guideline and/or historical patient data, outputting the result to an interpretability module and/or the diagnosis module.

4. The method according to claim 3, wherein the step of determining the relationship among named entities includes the step of expert supervision and/or annotation.

5. The method according to any one of claims 3 or 4, wherein an unsupervised transfer learning algorithm, such as Bidirectional Encoder Representations from Transformers, BERT, model, is used for the step mapping of the named entities to a clinical guideline and/or historical patient data.

6. The method according to any one of claims 1-5, wherein the step of mapping comprises applying a down-convolution Neural Network on the raw data, performing the mapping on the raw data, and applying an up-convolution Neural Network for receiving a fusion metric in a latent space, wherein the mapping comprises a feature map comprising named entities feature map and a relations feature map.

7. The method according to claim 6, wherein a conditional random field, CRF, probability classifier model and/or the LSTM model is applied to the fusion metric for extracting the context of the clinical information data.

8. The method according to any one of claims 1-7, wherein the clinical information data is configured to be recorded and converted into text by a voice recognition device.

9. The method according to any of claims 1-8, wherein the step of highlighting in the clinical report comprises highlighting the regions or objects of interest, comprising any one of: quality measures, Region of Interest, ROI, detection, fracture detection, lesion detection, visualization of dependencies between the radiology examination data and the clinical information data.

10. The method according to any of claims 1-10, wherein the method further comprises the step of adding additional information by a user to the clinical reports, which may comprise any one of: annotations, commenting, contouring.

11. The method according to any of claims 1-11, wherein the method further comprises step of collecting feedback in the clinical report comprises feedback from at least one node of the healthcare network: radiologist node, physician node, technician node, pathology node, clinical laboratory node, patient input node.

12. A method of training of data for the method of clinical decision support according to any of the claims 1-12, wherein the method of training comprises training the radiology examination data by a convolutional neural network, CNN, model and/or training the clinical information data by an unsupervised machine learning model.

13. The method according to claim 13, wherein the convolution neural network module is trained for providing clinical feedback to clinical nodes in the network.

14. A data processing system comprising a processor adapted to perform the steps of any of claims 1-13.

15. A computer program comprising instructions which, when the program is executed by a computer, cause the computer to carry out any of the method steps of claims 1-13.
